# EUROPEAN PATENT APPLICATION

(11) **EP 4 360 470 A1**
(43) Date of publication of application: **01.05.2024**
(21) Application number: 22209112.6
(22) Date of filing: 23.11.2022
(51) Int. Cl.: A23L 27/00, A61K 47/18

(54) **COMPOSITION AND METHOD FOR REDUCING NEGATIVE TASTE SENSATIONS OF PROPOLIS PREPARATIONS**

(30) Priority: 27.10.2022 GB 202215948
(71) Applicant: Dr Corbyn Ltd, Willington, County Durham DL15 0QL (GB); Smith, Alex-Fabian, Willington, County Durham DL15 0QL (GB)
(72) Inventor: Smith, Alex-Fabian, Willington, County Durham DL15 0QL (GB); Smith, Hannah, Willington, DL15 0QL (GB)

(57) **Abstract**

ABSTRACT

Composition and method for reducing negative taste sensations of propolis preparations

The present invention relates to a propolis composition and a method of using a propolis composition comprising at least one of a TRPA1 and/or TAS2R receptor antagonists to reduce/mask a subject's perception of the negative taste sensations of bitterness, astringency and pungency associated with propolis.

## Description

### TECHNICAL FIELD

The present invention relates to a propolis composition comprising at least one of a TRPA1 and/or TAS2R receptor antagonists to reduce/mask a subject's perception of the negative taste sensations of bitterness, astringency and pungency associated with propolis.

### BACKGROUND ART

Across the world, preparations containing propolis are used for a range of applications, most commonly in health supplements and oral preparations for human consumption. Propolis exhibits multiple biological functions, which include immunomodulatory, antioxidant, anti-inflammatory, anticancer, antibacterial, antifungal, antiviral, and antiparasitic functions (Wagh, 2013; Kocot et *al.,* 2018; Pobiega *et al*., 2019). Notable uses of propolis as reported in extant literature include: supporting immune function, reducing the inflammation and discomfort associated with a sore throat, lessening the duration of the common cold, and supporting oral health (Khayyal *et al*., 1993; Grange and Davey, 1990; Wagh, 2013; Wollenweber et *al.,* 1990).

However, due to the strong astringent, pungent and bitter taste of propolis, preparations containing propolis are not sensorily acceptable to customers, as highlighted by Rios et *al.* (2017). This reduces the commercial viability of the preparations, which is apparent from customer reviews of propolis-based products in general.

TABLE 1 below shows the most common terms utilised by customers with reference to their taste perceptions of liquid propolis preparations. A total of 2,450 customer reviews have been analysed, all of which refer to liquid propolis preparations currently marketed in various EU countries.

**TABLE 1**

| **The most common terms utilised by customers to describe their taste perceptions of propolis preparations currently marketed in the EU** | |
|---|---|
| 1. | Vile |
| 2. | Rancid |
| 3. | Bitter |
| 4. | Horrible |
| 5. | Disgusting |
| 6. | Horrific |
| 7. | Unpleasant |
| 8. | Acrid |
| 9. | Pungent |
| 10. | Foul |
| 11. | Horrid |
| 12. | Nasty |
| 13. | Bad |
| 14. | Revolting |
| 15. | Off-putting |
| 16. | Sickening |
| 17. | Repulsive |
| 18. | Gross |

It is well established that the bioactive composition of propolis will vary depending on its type, source, origin and age (Magnavacca *et al*., 2021; Wagh 2013). Over 300 constituents have been identified in different samples of propolis, which belong to the following groups of chemically similar compounds: polyphenols; benzoic acids and derivatives; cinnamic alcohol and cinnamic acid and its derivatives; sesquiterpene and triterpene hydrocarbons; benzaldehyde derivatives; other acids and respective derivatives; alcohols, ketones, and heteroaromatic compounds; terpene and sesquiterpene alcohols and theirderivatives; aliphatic hydrocarbons; minerals; sterols and steroid hydrocarbons; sugars and amino acids (Wagh, 2013; Marcucci, 1995; Park *et al.,* 2002; Pietta et *al.,* 2002; De Castro, 2001; Walker and Crane, 1987).

While the proportion of each propolis constituent is dependent on its type, source, origin and age, the presence of polyphenols in concentrations above 58% is consistently reported in most samples. (Kurek-Górecka et *al.,* 2014). High concentrations of polyphenols are associated with taste attributes of astringency and bitterness that decrease consumer acceptability, especially in children (Rios et *al.,* 2017). Further exploration of the literature indicates that the polyphenol content of propolis is positively related with taste attributes and may negatively influence the sensory acceptability of propolis preparations (Rios et *al.,* 2017).

Polyphenols are believed to activate TRPA1 receptors, which in turn induces taste sensations of pungency and astringency (Takahashi et *al.,* 2021). Further, it was discovered that various phenolic compounds act as TAS2R agonists, and as such will produce taste sensations of bitterness and astringency (Soares et *al.,* 2020). Different polyphenols activate different TAS2R receptors; for example, all phenolic acid ethyl esters activate TAS2R14 receptors, while vescalagin, castalagin, punicalagin, and grandinin activate the TAS2R7 receptor only (Soares et *al.,* 2018).

Therefore, what is needed is a composition that can reduce or mask the taste perceptions of pungency, astringency and bitterness associated with propolis by inhibiting activation of TRPA1 and TAS2R receptors, thus making the composition sensorily acceptable to customers as well as commercially viable to manufacturers. However, in selecting a suitable blocker to each of these receptors, it is necessary to consider its possible effects on the biological functions of propolis. Therefore, the selected blocker(s), or antagonist(s), should not alter the chemical structure of propolis, its biological functions or its therapeutic benefits.

### TECHNICAL PROBLEM

The inventors of the present invention have found that propolis preparations have a naturally bitter, astringent and pungent taste that is unpleasant to users of these preparations. These negative taste sensations are due to activation of TRPA1 and TAS2R taste receptors by the polyphenol constituents of propolis.

The present invention has been made in view of the above problems, and an object of the present invention is to reduce the negative taste sensations of pungency, astringency and bitterness associated with propolis preparations by inhibiting activation of TRPA1 and TAS2R taste receptors.

### SOLUTION TO PROBLEM

To overcome the problems described from Paragraphs 3 through 8, the present invention proposes a composition comprising at least one source of propolis (raw propolis, propolis extract, or combinations thereof) and at least one of: (i) an antagonist to TRPA1 receptor, (ii) an antagonist to TAS2R receptors, or (iii) combinations thereof.

### ADVANTAGEOUS EFFECTS OF THE INVENTION

According to the present invention, it is possible to reduce the negative taste perceptions of pungency, astringency and bitterness associated with propolis preparations, therefore making the preparations sensorily acceptable to users.

### DESCRIPTION OF EMBODIMENTS

It was found that antagonists to TRPA1 receptors provide a noticeable reduction in the level of perceived taste pungency and astringency when TRPA1 agonists are present. Further, it was discovered that antagonists to TAS2R receptors provide a noticeable reduction in the level of perceived taste bitterness and astringency when TAS2R agonists are present. Polyphenols have been identified as activators of TRPA1 and TAS2R receptors, thereby acting as agonists of these receptors. The presence of polyphenols in the bioactive composition of propolis means that TRPA1 and TAS2R receptors are activated when propolis preparations are used orally, which produce the negative taste sensations of pungency, bitterness and astringency associated with propolis. However, no antagonists to polyphenols have been found that act specific to the TPRA1 and TAS2R evoked taste sensations from propolis, either individually to each receptor or simultaneously across both receptors. Therefore, there is an unmet need to provide antagonists to these negative taste sensations of pungency, bitterness and astringency generated from the activation of TRPA1 and TAS2R receptors by the bioactive compounds of propolis, which is met by the present invention. While polyphenols act across the TRPA1 and TAS2R receptors simultaneously, it is not necessary to use an antagonist that blocks activation of the TRPA1 and TAS2R receptors simultaneously, orto use an antagonist for each receptor, in the same propolis preparation.

The negative sensorial attributes of propolis's activation of TRPA1 and TAS2R through means of its polyphenol content can be mitigated by adding an antagonist to propolis's activation to these receptors. Specifically, the present invention proposes a composition containing at least one source of propolis (raw propolis, propolis extract, or combinations thereof) and at least one of a TRPA1 and/or TAS2R receptor antagonist, which will block the activation of these receptors by the polyphenols contained in propolis and which will reduce or mask the perceived taste sensations of pungency, bitterness and astringency. The TRPA1 and/or TAS2R antagonist will be delivered at the same time as the agonist (polyphenols). The present invention relates to a composition containing >0.5% raw propolis, >0.1% propolis extract, or combinations thereof, and antagonists to the TRPA1 and/or TAS2R receptors.

Without being limited by theory, it is now believed that the negative taste sensations of pungency, bitterness and astringency produced by propolis activation of TRPA1 and TAS2R receptors, through means of propolis's polyphenol content, can be reduced or masked through the use of TRPA1 and TAS2R antagonists.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C (i.e. room temperature) unless otherwise specified.

As used herein, the word "about" means +/- 10 percent.

As used herein, the word "include," and its variants, are intended to be non-limiting, such that recitation of items in a list is not to the exclusion of other like items that may also be useful in the materials, compositions, devices, and methods of this invention.

As used herein, the word "or" when used as a connector of two or more elements is meant to include the elements individually and in combination; for example X or Y, means X or Y or both.

In accordance with the definition set out by the ISO 161 28-1:2016(E) International Standard, by "natural ingredient" is meant an ingredient that is obtained only from plants, animals, micro-organisms or minerals, including an ingredient that is obtained from these materials by:
- physical processes (e.g. grinding, drying, distillation),
- fermentation reactions occurring in nature and leading to molecules which occur in nature, and
- other procedures of preparation including traditional ones (e.g. extraction using solvents) without intentional chemical modification

By "propolis preparation" and "propolis composition" is meant a preparation containing at least one source of propolis (raw propolis, propolis extract or combinations thereof), which is intended for use in foods, health supplements, medicinal products, personal care products and/or oral care products to provide a beneficial effect. Non-limiting examples of propolis preparations include tinctures, sprays, syrups, lozenges, toothpastes, subgingival gels, mouth rinses, mousses, foams and breath-freshening products.

The propolis compositions according to the present invention comprise at least one source of propolis, which may either raw propolis, propolis extract or combinations thereof. Propolis preparations in general may be used for a wide range of applications with a view to providing a beneficial effect. Most of these preparations comprise a carrier material, for example water, propylene glycol, glycerol, vegetable glycerine or ethanol. However, solid propolis preparations may also be obtained, for example lozenges intended to relieve the discomfort associated with a sore throat. Blocking the activation of TRPA1 and TAS2R receptors generated by the bioactive compounds of propolis is desirable for both liquid and solid propolis preparations that are intended to be consumed orally or contacted with a body surface such as the mouth. This is met by the present invention. The compositions of the invention may typically comprise from about 0.5% to about 80% by weight of propolis source.

The propolis compositions of the invention may be formulated in any type of known chassis, such as a spray, syrup or lozenge.

The propolis compositions of the invention may comprise, in addition to the ingredients indicated above, additional ingredients in order to further enhance the properties of the compositions, including but not limited to: carrier materials, such as vegetable glycerine, propylene glycol and water; antioxidants; flavouring agents; agents for enhancing flavour or taste (e.g. monosodium glutamate, or MSG); natural ingredients; plant extracts; sweeteners; other bee products; and preserving agents.

The term "TRPA1 " or "TRPA1 receptor", as used herein, refers to the transient receptor potential cation channel, subfamily A, member 1, having a large cysteine-rich N-terminus that contains 18 predicted ankyrin repeats. TRPA1 is a ligand-gated, non-selective cation channel preferentially expressed on small diameter sensory neurons.

The term "TRPA1 agonist", as used herein, refers to any compound or substance that activates calcium release from intracellular stores through GPCR signalling and which increases one or more activities of the TRPA1 receptors by, for example, binding to, stimulating, increasing, opening, activating, facilitating, enhancing activating, sensitising, or upregulating TRPA1 receptor signal transduction. In the context of the TRPA1 receptor, the term "TRPA1 agonist" refers to a compound or substance that increases the downstream, signalling response associated with taste receptor TRPA1.

The terms "TRPA1 antagonist", "TRPA1 blocker" or "TRPA1 inhibitor", as used herein, refer to any compound or substance that decreases or blocks one or more activities of the TRPA1 taste receptor by, for example, binding to, partially or totally blocking stimulation, preventing, delaying activation, inactivating, desensitizing or down-regulating the TRPA1 taste receptor and/or taste transduction. A TRPA1 antagonist attenuates the effect of a TRPA1 agonist, such as polyphenols.

Wherein the TRPA1 antagonist may include one or more of the following: cinnamon bark oil; y -Dodecalactone; vanillic acid; y -Methyl Decalactone; trans, trans-2,4-Nonadienal; 4-Allyl-2,6-dimethoxyphenol; o-Methoxycinnamalde- hyde; 4-Methyl-2-phenyl-2 Pentenal (mix of cis and trans); 2-Methoxy-4-propyl-phenol; Methyl 2-methoxy-benzoate; γ- Tetradecalactone; 1-Methyl-2-pyrole carboxaldehyde; 3,3,5-Trimethylcyclohexanol; N-(2-Hydroxyethyl) lactamide; 2-(3-Phenylpropyl) tetrahydrofuran; Anisyl Butyrate; Methyl-4-phenyl butyrate; 3-Heptyldihydro-5-methyl-2(3H)-furanone; 3- acetylsulfanylhexyl acetate; 3-methyl-5-propyl-2-Cyclohexen-1-one; Isobornyl Isobutyrate; Bornyl Valerate; Citronellyl acetate; (2S,5S,6S)-6-) Hydroxydihydrotheaspirane; trans-2-Hexenal.

The term "TRPA1 enhancer", as used herein, refers to any compound that boosts the calcium flux activity of an agonist that directly activates TRPA1, but does not directly activate TRPA1.

The terms "TAS2R" or "TAS2R receptor", as used herein, refer to Taste Receptors Type 2, which is the family of 25 type A G protein coupled receptors (GPCRs) responsible for the perception of bittertaste in mammals. The terms "TAS2R" or "TAS2R receptor", as used herein, may refer to one or multiple bitter taste receptors. TAS2R receptors can be coupled to transducing or gustducin for example, through which they can activate both phospodiesterases and a phospholipase C (PLC) p2-dependent pathway to increase intracellular Ca2+ concentration.

The term "TAS2R agonist", as used herein, refers to any compound or substance that activates calcium release from intracellular stores through GPCR signalling and which increases one or more activities of any of the 25 TAS2R taste receptors by, for example, binding to, stimulating, increasing, opening, activating, facilitating, enhancing activating, sensitising, or upregulating bitter taste receptor signal transduction. In the context of bitter taste receptors TAS2R, the term "TAS2R agonist" refers to a compound or substance that increases the downstream, signalling response associated with the bitter taste receptors TAS2R.

The terms "TAS2R antagonist", "TAS2R blocker" or "TAS2R inhibitor", as used herein, refer to any compound or substance that decreases or blocks one or more activities of any of the TAS2R taste receptors by, for example, binding to, partially or totally blocking stimulation, preventing, delaying activation, inactivating, desensitizing or down-regulating the TAS2R taste receptors and/or taste transduction. A TAS2R antagonist attenuates the effect of a TAS2R agonist, such as polyphenols. In the context of bitter taste receptors TAS2R, the terms "TAS2R antagonist", "TAS2R blocker" or "TAS2R inhibitor" refer to a compound or substance that decreases the downstream, signalling response associated with the bitter taste receptors TAS2R. In certain embodiments, decreasing taste receptor activity can result in a change in the amount or distribution of an intracellular molecule or the activity of an enzyme which is part of the intracellular signalling pathway for the bitter receptor. Examples of the intracellular molecule include, but are not limited to, free calcium, cyclic adenosine monophosphate (cAMP), inositol mono-, di- ortri-phosphate. Examples ofthe enzyme include, but are not limited to, adenylate cyclase, phospholipase-C and G-protein coupled receptor kinase.

Wherein the TAS2R antagonist may include one or more of the following: Sucralose; Probenecid (p-(dipropylsulfamoyl) benzoic acid; SBA; 3-Hydroxypropionic acid (3HP); 1,5-anhydro-3-O-methyl-D-mannitol (3HD); Sodium saccharin; Aspartame; Sorbitol; Xylitol; Hardwickiic acid (as per WO2013072332A1 ); or combinations thereof.

It is desirable that propolis preparations for use in foods, health supplements, medicinal products, personal care products and/or oral care products do not produce taste sensations of bitterness, astringency or pungency. However, users also want to experience the therapeutic benefits imparted by propolis, thus they expect to experience negative taste sensations as an indicator of effectiveness, but at a level that is not perceived as overpowering. The ability to formulate a propolis preparation that is acceptable to users, however, raises challenges as the taste sensations produced by propolis are perceived as strong and overpowering. Thus, formulating user-acceptable propolis preparations can be a balancing act between an acceptable sensory experience and acceptable therapeutic benefits.

The first group of components which reduce the negative taste sensations of pungency and astringency associated with propolis are Transient Receptor Potential Ankryin 1 (TRPA1) antagonists. In looking at this receptor, it was discovered that combining antagonists of this receptor in the presence of agonists, such as polyphenols, caused a surprising effect. By adding a TRPA1 antagonist to a propolis composition, the user of the composition experiences reduced negative taste sensations and an improved perception as compared to a propolis composition without the TRPA1 antagonist. Thus, the TRPA1 antagonist is working to offset the negative taste sensations of pungency and astringency associated with propolis activation of TRPA1.

The second group of components which help to reduce the negative taste sensations of bitterness and astringency associated with propolis are Taste Receptors Type 2 antagonists. In looking at these receptors, it was discovered that combining antagonists of these receptors in the presence of agonists, such as polyphenols, caused a surprising effect. By adding a TAS2R antagonist to a propolis composition, the user of the composition experiences reduced negative taste sensations and an improved perception as compared to a propolis composition without the TAS2R antagonist. Thus, the TAS2R antagonist is working to offset the negative taste sensations of bitterness and astringency associated with propolis activation of TAS2R.

Further, where the agonist, such as polyphenols, targets both TRPA1 and TAS2R, applying antagonists to each receptor in the same preparation works synergistically to reduce the negative taste sensations generated by the agonist. A similar result is obtained by applying a single antagonist that blocks activation of TRPA1 and TAS2R simultaneously. However, it was found that a single antagonist to either the TRPA1 or TAS2R receptors will provide significant and sufficient reduction in the negative taste sensations.

In addition to the TRPA1 and TAS2R antagonists, the propolis compositions of the present invention may also comprise one or more of the following components: carrier materials, such as vegetable glycerine, propylene glycol and water; antioxidants; flavouring agents; agents for enhancing flavour or taste (e.g. monosodium glutamate, or MSG); natural ingredients; plant extracts; sweeteners; other bee products; and preserving agents.

Actives and other ingredients may be categorised or described herein by their therapeutic benefit, function or their postulated mode of action. However, it is to be understood that the active and other ingredients useful herein can, in some instances, provide morethan onetherapeutic benefit, function, or can operate via more than one mode of action. Therefore, classifications herein are made for the sake of convenience and are not intended to limit an ingredient to the particularly stated function(s) or activities listed.

Carrier materials include water, glycerine, sorbitol, polyethylene glycols having a molecular weight of less than about 50,000, propylene glycol and other edible polyhydric alcohols, ethanol, or combinations thereof. The propolis compositions of the present invention include from about <5% to about 93% of a carrier material by weight of the preparation. In certain embodiments, the compositions contain carrier materials in an amount of from about 10% to about 60% by total weight of the propolis preparation.

Antioxidants that are approved in the UK for use in food, according to the Food Standards Agency, include: E300 Ascorbic acid; E301 Sodium ascorbate; E302 Calcium ascorbate; E304 Fatty acid esters of ascorbic acid; E306 Tocopherols; E307 Alpha-tocopherol; E308 Gamma-tocopherol; E309 Delta-tocopherol; E310 Propyl gallate; E315 Erythorbic acid; E316 Sodium erythorbate; E319 Tertiary-butyl hydroquinone (TBHQ); E320 Butylated hydroxyanisole (BHA); E321 Butylated hydroxytoluene (BHT); E392 Extracts of rosemary; E5864-Hexylresorcinol; and combinations thereof.

While most propolis compositions of the present invention do not comprise antioxidants, in some embodiments these may be present in an amount of from about 0.05% to about 5% by total weight of the propolis composition.

Examples of some flavours and flavour components that may be used in propolis compositions are mint oils, lemon, orange, honey, propenyl guaethol, heliotropine, 4-cis-heptenal, diacetyl, methyl-p-tert-butyl phenyl acetate, methyl salicylate, ethyl salicylate, 1-menthyl acetate, oxanone, γ - irisone, methyl cinnamate, ethyl cinnamate, butyl cinnamate, ethyl butyrate, ethyl acetate, methyl anthranilate, iso-amyl acetate, iso-amyl butyrate, allyl caproate, eugenol, eucalyptol, thymol, cinnamic alcohol, octanol, octanal, decanol, decanal, phenylethyl alcohol, benzyl alcohol, γ-terpineol, linalool, limonene, citral, neral, geranial, geraniol nerol, maltol, ethyl maltol, anethole, dihydroanethole, carvone, menthone, y -damascenone, ionone, y -decalactone, γ - nonalactone, □-undecalactone, or combinations thereof. Generally suitable flavouring ingredients are chemicals with structural features similar to those of the main carrier material utilised in formulating the propolis preparations of the present invention. Here, it is desirable (i) that the flavouring ingredient and main carrier material are miscible and dissolve in each other to form a homogenous solution without separation, or (ii) that the flavouring ingredient is soluble in the main carrier material. These flavouring ingredients include derivatives of flavour chemicals that are saturated or contain stable aromatic rings or ester groups. For example, where the propolis preparation comprises propylene glycol as the main carrier material, an oilbased flavouring ingredient is not suitable as propylene glycol and oil are not miscible and oil will separate from the propylene glycol. In such embodiments, suitable flavouring ingredients are those that are also in a propylene glycol base, or in an alcohol or water base as both alcohol and water are soluble in propylene glycol.

Flavours are generally present in an amount of from about 0.3% to about 5% or from about 1% to about 3% by total weight of the propolis composition.

Agents for enhancing flavours, or flavour enhancers, are substances used to intensify flavours. Monosodium glutamate (MSG) is one of several forms of glutamic acid found in foods and also the most commonly used flavour enhancer in the food industry.

While most propolis compositions of the present invention do not comprise flavour enhancers, in some embodiments these may be present in an amount of from about 0.04% to about 2% by total weight of the propolis composition.

Natural ingredients include: plant and fruit powders; certain essential oils; bee products; plant parts, such as the roots, rhizomes and aerial parts of plants; cold-pressed unrefined oils, such as hemp seed oil and coconut oil; plant-derived substances, such as the gel from the leaves of the Aloe barbadensis (aloe vera) plant; humectants, such as vegetable glycerine; and combinations thereof.

In certain embodiments of the present invention, natural ingredients may be present in an amount of from about 1% to about 30% by total weight of the propolis composition.

Plant extracts include extracts of: Sambucus (elderberry); Echinacea purpurea or Echinacea angustifolia (echinacea); Camellia sinensis (green tea); Zingiber officinale (ginger); Nigella sativa (black cumin); Glycyrrhiza glabra (licorice); Panax L. (American ginseng); Astragalus membranaceus (Astragalus); Salvia officinalis (sage); Matricaria chamomilla (chamomile); Rosmarinus officinalis (rosemary); Melissa officinalis (lemon balm); Origanum vulgare (oregano); Petroselinum crispum (parsley); and combinations thereof. This list is not exhaustive. Such plant extracts may be included in the formulation of propolis compositions to enhance the therapeutic benefits of propolis.

While most propolis compositions of the present invention do not comprise plant extracts, in some embodiments these may be present in an amount of from about 2% to about 30% by total weight of the propolis composition.

Other bee products include honey, pollen, royal jelly, and combinations thereof.

Other bee products are generally present in an amount of from about 0.05% to about 60% by total weight of the propolis composition.

Sweeteners include: saccharin; chloro-sucrose (sucralose); steviolglycosides; rebaudioside A; rebaudioside B; rebaudioside C; rebaudioside D; rebaudioside E; rebaudioside F; dulcoside A; dulcoside B; rubusoside; stevia; stevioside; acesulfame K; xylitol; neohesperidine DC; alitame; aspartame; neotame; alitame; thaumatin; cyclamate; glycyrrhizin; mogroside IV; mogroside V; Luo Han Guo sweetener; siamenoside; monatin and its salts (monatin SS; RR; RS; SR); curculin; monellin; mabinlin; brazzein; hemandulcin; phyllodulcin; glycyphyllin; phloridzin; trilobatin; baiyanoside; osladin; polypodoside A; pterocaryoside A; pterocaryoside B; mukurozioside; phlomisoside I; periandrin I; abrusoside A; cy- clocarioside I;N-[N-[3-(3-hydroxy-4-methoxyphenyl)propyl]-L-y -aspartyl]-L-phenylalanine 1-methyl ester; N-[N-[3-(3-hy- droxy-4-methoxyphenyl)-3-methylbutyl]-L-y -aspartyl]-L-phenylalanine 1-methyl ester; N-[N-[3-(3-methoxy-4-hydroxy-phenyl)propyl]-L- y -aspartyl]-L-phenylalanine 1-methyl ester; salts thereof; and combinations thereof.

Preserving agents (or preservatives) that are approved for use in food in the UK, according to the Food Standards Agency, include: E200 Sorbic acid; E202 Potassium sorbate; E210 Benzoic acid; E211 Sodium benzoate; E212 Potassium benzoate; E213 Calcium benzoate; E214 Ethyl p-hydroxybenzoate; E215 Sodium ethyl p-hydroxybenzoate; E218 Methyl p-hydroxybenzoate; E219 Sodium methyl p-hydroxybenzoate; E220 Sulphur dioxide; E221 Sodium sulphite; E222 Sodium hydrogen sulphite; E223 Sodium metabisulphite; E224 Potassium metabisulphite; E226 Calcium sulphite; E227 Calcium hydrogen sulphite; E228 Potassium hydrogen sulphite; E234 Nisin; E235 Natamycin; E239 Hexamethylenetetramine; E242 Dimethyl decarbonate; E243 Ethyl lauroyl arginate; E249 Potassium nitrite; E250 Sodium nitrite; E251 Sodium nitrate; E252 Potassium nitrate; E280 Propionic acid; E281 Sodium propionate; E282 Calcium propionate; E283 Potassium propionate; E284 Boric acid; E285 Sodium tetraborate, borax; E1105 Lysozyme; and combinations thereof.

While most propolis compositions of the present invention do not comprise preservatives, in some embodiments these may be present in an amount of from about 0.05% to about 1.5% by total weight of the propolis composition.

### EXAMPLES

### EXAMPLE 1

The first group of components which will help to reduce the negative taste sensations of pungency and astringency associated with propolis are Transient Receptor Potential Ankryin 1 (TRPA1 ) antagonists. In looking at this receptor, it was discovered that combining antagonists of this receptor in the presence of polyphenols caused a surprising effect. By adding a TRPA1 antagonist to a propolis composition, the user of the composition experienced reduced negative taste sensations and an improved perception and in-use experience of the composition, over a composition without the TRPA1 antagonist. Thus, the TRPA1 antagonist is working to offset the negative taste sensations of pungency and astringency associated with propolis activation of TRPA1.

The compounds listed in TABLE 2 below are known to be antagonists of the TRPA1 receptor as shown in EP2998001A1, in that they reduce the level of TRPA1 receptor activation when activated by agonists such as polyphenols. Only 20 compounds have been listed, however, it is to be understood that the list is not exhaustive.

**TABLE 2**

| **Compound (which is a TRPA1 antagonist)** | |
|---|---|
| 1. | Cinnamon bark oil |
| 2. | Vanillic acid |
| 3. | γ -Methyl decalactone |
| 4. | Piperazine |
| 5. | Manganese chloride |
| 6. | Desoxycholic acid |
| 7. | Sodium erythorbate |
| 8. | DI-verbenone |
| 9. | 3-hydroxybenzoic acid |
| 10. | Ferric chloride |
| 11. | Phenylethyl isovalerate |
| 12. | Methyl linoleate |
| 13. | Isobutyl isobutyrate |
| 14. | Quinoline |
| 15. | 1-undecanol |
| 16. | 1-stearoyl-rac-glycerol |
| 17. | 2-undecanol |
| 18. | Phloretin |
| 19. | 1,2-propanedithiol |
| 20. | y -Dodecalactone |

### EXAMPLE 2

The second group of components tested for their ability to reduce the negative taste sensations of bitterness and astringency associated with propolis are Taste Receptors Type 2 (TAS2R) antagonists. In looking at these receptors, it was discovered that combining antagonists of these receptors in the presence of agonists, such as polyphenols, caused a surprising effect. By adding a TAS2R antagonist to a propolis composition, the user of the composition experienced reduced negative taste sensations and an improved perception and in-use experience of the composition, over a composition without the TAS2R antagonist. Thus, the TAS2R antagonist is working to offset the negative taste sensations of bitterness and astringency associated with propolis activation of TAS2R.

The compounds listed in TABLE 3 below are known to be antagonists of various TAS2R receptors, in that they reduce the level of TAS2R receptor activation when activated by agonists such as polyphenols. Only 10 compounds have been listed, however, it is to be understood that the list is not exhaustive.

**TABLE 3**

| **Compound (which is a TAS2R antagonist)** | |
|---|---|
| 1. | Sucralose |
| 2. | Probenecid (p-(dipropylsulfamoyl) |
| | benzoic acid |
| 3. | SBA |
| 4. | 3-Hydroxypropionic acid (3HP) |
| 5. | 1,5-anhydro-3-O-methyl-D-mannitol (3HD) |
| 6. | Sodium saccharin |
| 7. | Aspartame |
| 8. | Sorbitol |
| 9. | Xylitol |
| 10. | Hardwickiic acid (as per WO2013072332A1) |

While any TRPA1 receptor antagonist may be utilised in the compositions of the present invention to reduce or mask the negative taste sensations of pungency and astringency associated with propolis activation of this receptor, it was found that vanillic acid (4-hydroxy-3-methoxybenzoic acid) provides a more balanced and consistent reduction in these negative taste sensations. However, it is to be understood that the selection of a TRPA1 receptor antagonist is not limited to vanillic acid only, and that other compounds with TRPA 1 blocking activity, either individually or in combination, may also be used as appropriate.

### EXAMPLE 3

For EXAMPLES 3 to 8, sensory evaluation studies were conducted using a methodology patterned after the techniques described in Meilgaard et *al.* (2007). Panels of trained sensory experts evaluated the taste sensations experienced after using a propolis composition with or without the TRPA1 antagonist, as described herein.

For EXAMPLE 3, a panel of 17 trained sensory experts evaluated the sensory profile experienced after application of the propolis composition sample formulations shown in TABLE 4 below, followed by rinsing with water.

The propolis composition sample formulations are shown below in TABLE 4. These compositions were formulated using conventional methods and are shown below with component amounts in weight % of total composition.

TABLE 4 contains the formulations for propolis compositions with propolis content going from 0.5% to 60% (Samples A-J, TABLE 4). The sample formulations were prepared either with propylene glycol and water (samples A, C, E, G and I), or with ethanol and water (samples B, D, F, H and J).

**TABLE 4**

| **Ingredient** | **A** | **B** | **C** | **D** | **E** | **F** | **G** | **H** | **I** | **J** |
|---|---|---|---|---|---|---|---|---|---|---|
| **Propolis** | 0.5% | 0.5% | 5% | 5% | 15% | 15% | 35% | 35% | 60% | 60% |
| **Propylene glycol** | 96.5% | 0% | 93% | 0% | 83% | 0% | 65% | 0% | 40% | 0% |
| **Water** | 3% | 3% | 2% | 2% | 2% | 2% | 0% | 0% | 0% | 0% |
| **Ethanol** | 0% | 96.5% | 0% | 93% | 0% | 83% | 0% | 65% | 0% | 40% |

Seventeen trained panelists separately applied 3 sprays (0.45ml) of each propolis composition (A-J) to the back of their throat and rated them separately according to:
- the perceived level of taste pungency of the composition by assigning a number between 0 ('Not Pungent') and 10 ('Very Pungent')
- the perceived level of taste astringency of the composition by assigning a number between 0 ('Not Astringent') and 10 ('Very Astringent')

They then rinsed their mouth with 20ml of tap water at room temperature (average temperature of 20°C). Panelists assessed the perceived levels of taste astringency and pungency according to the same two 10-point scales at 0 minutes after rinse expectoration, then again 5 minutes, 15 minutes, 30 minutes and 45 minutes after rinse expectoration. At each evaluation, they assessed each taste sensation separately on the 1 0-point scale. Further, at each evaluation, panelists were instructed to breathe in and out, and then to evaluate the overall taste sensations of pungency and astringency. Results are shown in TABLES 5 and 6 below.

**TABLE 5**

| | **Perceived level of taste pungency** | | | | | | | | | |
|---|---|---|---|---|---|---|---|---|---|---|
| **Sample** | **A** | **B** | **C** | **D** | **E** | **F** | **G** | **H** | **I** | **J** |
| **At application** | 4.2 | 4.5 | 7.4 | 7.8 | 8.5 | 8.3 | 8.7 | 8.7 | 9.1 | 9.8 |
| **0 mins after rinse** | 4.0 | 4.2 | 7.1 | 7.4 | 8.3 | 8.0 | 8.1 | 8.3 | 9.0 | 9.4 |
| **5 mins after rinse** | 3.6 | 3.9 | 6.6 | 7.1 | 7.8 | 7.9 | 7.5 | 7.9 | 8.7 | 9.1 |
| **15 mins after rinse** | 3.2 | 3.7 | 6.3 | 6.7 | 7.1 | 7.4 | 7.2 | 7.7 | 8.2 | 8.7 |
| **30 mins after rinse** | 2.9 | 3.1 | 5.9 | 6.1 | 6.9 | 7.1 | 7.0 | 7.2 | 7.8 | 8.4 |
| **45 mins after rinse** | 2.9 | 2.9 | 5.4 | 4.9 | 6.5 | 6.9 | 6.8 | 6.9 | 7.5 | 7.7 |

**TABLE 6**

| | **Perceived level of taste astringency** | | | | | | | | | |
|---|---|---|---|---|---|---|---|---|---|---|
| **Sample** | **A** | **B** | **C** | **D** | **E** | **F** | **G** | **H** | **I** | **J** |
| **At application** | 3.2 | 3.5 | 5.5 | 5.4 | 6.7 | 6.6 | 7.9 | 8.1 | 8.5 | 8.2 |
| **0 mins after rinse** | 3.0 | 3.3 | 5.1 | 5.1 | 6.4 | 6.5 | 7.8 | 7.9 | 8.1 | 7.9 |
| **5 mins after rinse** | 2.8 | 2.9 | 4.9 | 4.7 | 6.2 | 6.0 | 7.6 | 7.7 | 7.7 | 7.4 |
| **15 mins after rinse** | 2.7 | 2.4 | 4.9 | 4.5 | 6.0 | 5.6 | 7.0 | 7.2 | 7.4 | 7.3 |
| **30 mins after rinse** | 2.3 | 2.0 | 4.2 | 4.2 | 5.7 | 5.5 | 6.6 | 6.7 | 7.0 | 6.8 |
| **45 mins after rinse** | 2.1 | 1.9 | 3.4 | 3.8 | 5.3 | 5.2 | 5.9 | 5.4 | 6.2 | 6.5 |

As shown in TABLES 5 and 6, the perceived intensity of both taste sensations increases proportionally with the concentration of propolis. The perceived taste sensations did not reduce significantly 0 minutes after panelists rinsed their mouth with water. A significant reduction was noted after the 30-minute mark.

### EXAMPLE 4

For TABLE 7, an evaluation of the perceived effectiveness of the sample formulations A to J was conducted. The same panel of trained sensory experts (as per TABLES 5 and 6), who at the time reported discomfort due to cold symptoms including a sore throat, separately applied 3 sprays (0.45ml) of each propolis composition (A-J) to the back of theirthroat a second time and rated them on a 1 0-point scale according to the perceived level of effectiveness for improving symptoms of a sore throat, from 0 ('Not Effective') to 10 ('Very Effective'). Evaluations were conducted 30 minutes and 1 hour after application. In this test, a numerical score between 3.5 and 6.5 indicates an acceptable level of perceived effectiveness, which is not statistically significant. Numerical scores above 6.5 indicate that the level of perceived effectiveness is high, whereas scores below 3.5 suggest a low level of perceived effectiveness. Results are shown in TABLE 7 below.

**TABLE 7**

| | **Perceived level of effectiveness for improving symptoms of a sore throat** | | | | | | | | | |
|---|---|---|---|---|---|---|---|---|---|---|
| **Sample** | **A** | **B** | **C** | **D** | **E** | **F** | **G** | **H** | **I** | **J** |
| **30 minutes after application** | 6.1 | 6.0 | 7.6 | 7.7 | 8.1 | 8.1 | 8.6 | 8.4 | 8.9 | 8.9 |
| **1 hour after application** | 7.2 | 6.9 | 8.5 | 8.9 | 9.3 | 9.6 | 9.7 | 9.6 | 9.7 | 9.5 |

For samples comprising <0.5% propolis, the scores were below 6.5 30 minutes after application. However, these increased to above 6.5 after 1 hour. All other scores reported by the panelists in TABLE 7 are above 6.5, which indicates that the perceived level of effectiveness is high in general and not negatively impacted by the perceived taste sensations of pungency and astringency. As such, the sensory acceptability of the samples was not correlated to their perceived effectiveness; even if the taste was not found to be acceptable to panelists, this did not modulate their perceptions of effectiveness. Nonetheless, the lack of sensory acceptability will reduce the commercial viability of propolis compositions.

### EXAMPLE 5

For TABLE 8, a panel of 13 trained sensory experts evaluated the taste acceptability of the compositions A to J on the 9-point hedonic taste scale developed by the Quartermaster Food and Container Institute of the U.S. Armed Forces. Panelists applied 3 sprays (0.45ml) of each composition (A-J) to the back of their throat and rated each sample separately, from 1 ('Dislike Extremely') to 9 ('Like Extremely') according to their personal perception of the taste acceptability of each sample. A single evaluation was conducted, which was immediately after product application. Results are shown in TABLE 8 below.

**TABLE 8**

| | **Perceived taste acceptability based on the 9-point hedonic taste scale** | | | | | | | | | |
|---|---|---|---|---|---|---|---|---|---|---|
| **Sample** | **A** | **B** | **C** | **D** | **E** | **F** | **G** | **H** | **I** | **J** |
| **After application** | 4.0 | 4.2 | 3.0 | 3.1 | 1.8 | 1.5 | 1.2 | 1.4 | 1.0 | 1.1 |

The data shown in TABLE 8 above indicate a low level of perceived taste acceptability for propolis compositions comprising >0.5% propolis without a TRPA1 antagonist, with an average score of 2.2. For compositions comprising >0.5% propolis (samples C to J), all scores in TABLE 8 are at or below the 3-point mark, which is 'Dislike Moderately'. For samples containing propolis concentrations between 15% and 60% by total weight of the composition, scores were at or below the 2-point mark, which is 'Dislike Very Much'. For samples containing propolis concentrations >60% by total weight of the composition, scores were at the 1-point mark ('Dislike Extremely'), indicating the lowest taste acceptability. These results show that propolis compositions have low user acceptability due to their poor sensory profile, in line with extant research, which is likely to negatively impact their commercial viability. The only samples with a score higher than 3 were samples A and B, which had a very low propolis concentration of 0.5% by weight of the composition. However, propolis preparations in general comprise >5% propolis by total weight of the composition to provide a beneficial effect, and as such lower concentrations are unlikely to be placed on the market despite having a slightly more acceptable sensory profile.

TABLES 5, 6, 7 and 8 demonstrate the need to add pungent and astringent taste antagonists to propolis compositions comprising >0.5% propolis by total weight of the composition.

### EXAMPLE 6

For EXAMPLE 6, all samples A1 to J10 had the same formulation as samples A to J (TABLE 4) but with the addition of a TRPA1 antagonist. Each sample A1 to J10 had vanillic acid added at 0.5% by total weight of the composition.

A panel of 29 trained sensory experts separately applied 3 sprays (0.45ml) of each propolis composition containing the TRPA1 antagonist (A1-J10) to the back of their throat and rated them separately according to:
- the perceived level of taste pungency of the composition by assigning a number between 0 ('Not Pungent') and 10 ('Very Pungent')
- the perceived level of taste astringency of the composition by assigning a number between 0 ('Not Astringent') and 10 ('Very Astringent')

They then rinsed their mouth with 20ml of tap water at room temperature (average temperature of 20°C). Panelists assessed the perceived levels of taste astringency and pungency according to the same two 10-point scales at 0 minutes after rinse expectoration, then again 5 minutes, 15 minutes, 30 minutes and 45 minutes after rinse expectoration. At each evaluation, they assessed each taste sensation separately on the 1 0-point scale. Further, at each evaluation, panelists were instructed to breathe in and out, and then to evaluate the overall taste sensations of pungency and astringency. In this test, a numerical score between 4.0 and 7.0 indicates a noticeable trend in the specified parameter, which is not statistically significant, but noticeable. Numerical scores under 4.0 indicate significant differences or definite reductions in the perceived levels of taste pungency and astringency. Results for both taste sensations are reported in TABLES 9 and 10 below.

**TABLE 9**

| | **Perceived level of taste pungency for samples containing a TRPA1 antagonist (0.5% vanillic acid)** | | | | | | | | | |
|---|---|---|---|---|---|---|---|---|---|---|
| **Sample** | **A1** | **B2** | **C3** | **D4** | **E5** | **F6** | **G7** | **H8** | **I9** | **J10** |
| **At application** | 2.2 | 2.1 | 2.8 | 2.8 | 3.1 | 3.0 | 3.8 | 3.9 | 4.5 | 4.4 |
| **0 mins after rinse** | 2.0 | 1.9 | 2.5 | 2.6 | 3.0 | 2.8 | 3.6 | 3.6 | 4.0 | 4.0 |
| **5 mins after rinse** | 1.6 | 1.6 | 2.1 | 2.4 | 2.8 | 2.5 | 3.1 | 3.3 | 3.7 | 3.8 |
| **15 mins after rinse** | 1.4 | 1.2 | 1.9 | 2.0 | 2.4 | 2.4 | 2.8 | 2.9 | 3.3 | 3.1 |
| **30 mins after rinse** | 1.2 | 1.0 | 1.6 | 1.9 | 2.0 | 2.1 | 2.5 | 2.5 | 2.8 | 2.9 |
| **45 mins after rinse** | 0.5 | 0.7 | 1.1 | 1.4 | 1.5 | 1.8 | 2.0 | 2.1 | 2.4 | 2.2 |

**TABLE 10**

| | **Perceived level of taste astringency for samples containing a TRPA1 antagonist (0.5% vanillic acid)** | | | | | | | | | |
|---|---|---|---|---|---|---|---|---|---|---|
| **Sample** | **A1** | **B2** | **C3** | **D4** | **E5** | **F6** | **G7** | **H8** | **I9** | **J10** |
| **At application** | 1.2 | 1.1 | 2.7 | 2.8 | 3.1 | 3.1 | 3.8 | 3.9 | 4.0 | 4.4 |
| **0 mins after rinse** | 1.0 | 0.9 | 2.1 | 2.5 | 2.8 | 2.6 | 3.4 | 3.4 | 3.7 | 4.0 |
| **5 mins after rinse** | 1.0 | 0.9 | 2.0 | 2.1 | 2.7 | 2.3 | 3.0 | 3.1 | 3.4 | 3.2 |
| **15 mins after rinse** | 1.0 | 0.5 | 1.7 | 1.9 | 2.3 | 2.0 | 2.8 | 2.7 | 3.0 | 3.0 |
| **30 mins after rinse** | 0.7 | 0.4 | 1.2 | 1.4 | 2.0 | 1.8 | 2.3 | 2.3 | 2.8 | 2.9 |
| **45 mins after rinse** | 0.5 | 0 | 0.8 | 1.1 | 1.6 | 1.2 | 1.9 | 1.8 | 2.1 | 2.3 |

As shown in TABLES 9 and 10, the taste sensations of pungency and astringency are significantly reduced when a TRPA1 antagonist (0.5% vanillic acid) is added. The reduction was noticeable across all sample formulations A1 to J1 0 and for both carrier materials used (propylene glycol and ethanol).

### EXAMPLE 7

ForTABLE 11, an evaluation of the perceived effectiveness of the sample formulations containing 0.5% vanillic acid was conducted. The same panelists (as per TABLES 9 and 10), who at the time reported discomfort due to cold symptoms including a sore throat, separately applied 3 sprays (0.45ml) of each propolis composition (A1-J10) to the back of their throat and rated them on a 10-point scale according to the perceived level of effectiveness, from 0 ('Not Effective') to 10 ('Very Effective'). Evaluations were conducted 30 minutes and 1 hour after application. In this test, a numerical score between 3.5 and 6.5 indicates an acceptable level of perceived effectiveness, which is not statistically significant. Numerical scores above 6.5 indicate that the level of perceived effectiveness is high, therefore reducing the taste sensations of pungency and astringency does not impact users' perceived effectiveness of a propolis composition containing a TRPA1 antagonist for improving the symptoms of a sore throat. Results are shown in TABLE 11 below.

**TABLE 11**

| | **Perceived level of effectiveness for improving symptoms of a sore throat for samples containing a TRPA1 antagonist (0.5% vanillic acid)** | | | | | | | | | |
|---|---|---|---|---|---|---|---|---|---|---|
| **Sample** | **A** | **B** | **C** | **D** | **E** | **F** | **G** | **H** | **I** | **J** |
| **30 minutes after application** | 6.4 | 6.3 | 7.9 | 8.2 | 8.8 | 8.1 | 8.4 | 8.7 | 9.1 | 9.0 |
| **1 hour after application** | 7.4 | 7.1 | 8.7 | 8.7 | 9.1 | 9.5 | 9.6 | 9.4 | 9.5 | 9.5 |

For samples comprising >0.5% propolis by total weight of the composition (Samples C to J), all scores reported by the panelists in TABLE 11 are above 6.5, which indicates that users' perception of the effectiveness of a propolis composition is not negatively impacted by reduced negative taste sensations of bitterness and astringency. There is not a significant difference between the samples containing a TRPA1 antagonist and the samples without a TRPA1 antagonist. Therefore, the user acceptability and commercial viability are not negatively impacted when the taste sensations of pungency and astringency are reduced. A slight improvement in the sensory profile of samples comprising <0.5% propolis by total weight of the composition (Samples A and B) was also noted.

### EXAMPLE 8

For EXAMPLE 8, a panel of 13 trained sensory experts (as per TABLE 8) evaluated the taste acceptability of the sample compositions (A1 to J10) containing TRPA1 antagonist 0.5% vanillic acid on the 9-point hedonic taste scale developed by the Quartermaster Food and Container Institute of the U.S. Armed Forces. Panelists applied 3 sprays (0.45ml) of each propolis composition (A1-J10) to the back of their throat and rated each sample separately, from 1 ('Dislike Extremely') to 9 ('Like Extremely') according to their personal perception of the taste acceptability of each sample. A single evaluation was conducted after application. Results are shown in TABLE 12 below.

**TABLE 12**

| | **Perceived taste acceptability for samples containing a TRPA1 antagonist (0.5% vanillic acid) based on the 9-point hedonic taste scale** | | | | | | | | | |
|---|---|---|---|---|---|---|---|---|---|---|
| **Sample** | **A1** | **B2** | **C3** | **D4** | **E5** | **F6** | **G7** | **H8** | **I9** | **J10** |
| **After application** | 8.2 | 8.0 | 7.8 | 7.5 | 7.1 | 7.0 | 6.5 | 6.7 | 6.2 | 6.0 |

The data shown in TABLE 12 above indicate a considerable improvement in the level of perceived taste acceptability for propolis compositions containing a TRPA1 antagonist (0.5% vanillic acid). All scores were above the 6-point mark, which is 'Like Slightly'. For samples containing lower concentrations of propolis (<15% by total weight of the composition), scores were above the 7-point mark, which is 'Like Moderately'.

The results show that the addition of a TRPA1 antagonist when a TRPA1 agonist is present (polyphenols) will significantly improve the taste acceptability of propolis compositions, by 293% on average. The overall user acceptability and commercial viability of the propolis compositions of the present invention will therefore be significantly higher compared to compositions without a TRPA1 antagonist.

TABLES 9 and 10 demonstrate that the present invention provides a practical and effective solution to improving the sensory acceptability of propolis compositions, thus increasing commercial viability. As was posited previously, the addition of a TRPA1 antagonist in the presence of a TRPA1 agonist (polyphenols) will reduce the perceived taste sensations of pungency and astringency associated with propolis activation of this receptor without a negative impact on perceived effectiveness. TABLE 12 shows an average improvement of 293% in user taste acceptability when a TRPA1 antagonist is added compared to the samples without the antagonist. Further, TABLE 11 shows that the perceptions of effectiveness of propolis compositions of the present invention are not negatively impacted by a reduction in negative taste sensations. As such, the addition of a TRPA1 antagonist to a propolis composition will benefit its user acceptability and commercial viability without diminishing its perceived therapeutic benefits.

### EXAMPLE 9

Sweeteners, as listed in Paragraph 51 and TABLE 3 above, have been found to act as TAS2R antagonists, in that they reduce the taste sensations of bitterness and astringency associated with propolis activation of TAS2R. Further, sweeteners act as agonists of Taste Receptors Type 1 (TAS1R) receptors in that they activate TAS1R receptors to produce taste sensations of sweetness. Therefore, the addition of at least one sweetener, which acts as a TAS2R antagonist and a TAS1R agonist, reduces/masks the taste sensations of bitterness and astringency and imparts a sweet taste to the user of the propolis composition of the present invention.

As demonstrated in EXAMPLES 10 to 15 described below, the negative taste sensations produced by propolis, and specifically by its polyphenol content, can be reduced using at least one TAS2R antagonist. These reductions translate into a user noticeable signal from the propolis composition. For the propolis compositions of the present invention, sweeteners were found to be more suitable as both TAS2R antagonists and TAS1R agonists. However, other TAS2R antagonist may be used also.

For EXAMPLES 10 to 15, sensory evaluation studies were conducted using a methodology patterned after the techniques described in Meilgaard et *al.* (2007). Panels of trained sensory experts evaluated the taste sensations experienced after using a propolis composition with or without the TAS2R antagonist, as described herein.

### EXAMPLE 10

For EXAMPLE 10, a panel of 22 trained sensory experts evaluated the sensory profile experienced after application of the propolis composition sample formulations shown in TABLE 13 below, followed by rinsing with water.

The propolis composition sample formulations are shown below in TABLE 13. These compositions were formulated using conventional methods and are shown below with component amounts in weight % of total composition.

TABLE 13 contains the formulations for propolis compositions with propolis content going from 5% to 60% (Samples K-P, TABLE 13). The sample formulations were prepared either with propylene glycol and water(samples K, M and O), or with ethanol and water (samples L, N and P).

**TABLE 13**

| **Ingredient** | **K** | **L** | **M** | **N** | **O** | **P** |
|---|---|---|---|---|---|---|
| **Propolis** | 5% | 5% | 15% | 15% | 60% | 60% |
| **Propylene glycol** | 93% | 0% | 83% | 0% | 40% | 0% |
| **Water** | 2% | 2% | 2% | 2% | 0% | 0% |
| **Ethanol** | 0% | 93% | 0% | 83% | 0% | 40% |

Twenty-two trained panelists separately applied 3 sprays (0.45ml) of each propolis composition (K-P) to the back of their throat and rated them separately according to:
- the perceived level oftaste bitterness ofthe preparation by assigning a number between 0 ('Not Bitter') and 10 ('Very Bitter')
- the perceived level of taste astringency of the preparation by assigning a number between 0 ('Not Astringent') and 10 ('Very Astringent')

They then rinsed their mouth with 20ml of tap water at room temperature (average temperature of 20°C). Panelists assessed the perceived levels of taste astringency and bitterness according to the same two 10-point scales at 0 minutes after rinse expectoration, then again 5 minutes, 15 minutes, 30 minutes and 45 minutes after rinse expectoration. At each evaluation, they assessed each taste sensation separately on the 1 0-point scale. Further, at each evaluation, panelists were instructed to breathe in and out, and then to evaluate the overall taste sensations of bitterness and astringency. Results are shown in TABLES 14 and 15 below.

**TABLE 14**

| | **Perceived level of taste bitterness** | | | | | |
|---|---|---|---|---|---|---|
| **Sample** | **K** | **L** | **M** | **N** | **O** | **P** |
| **At application** | 7.7 | 8.1 | 8.2 | 8.4 | 9.2 | 9.7 |
| **0 mins after rinse** | 6.2 | 7.8 | 8.0 | 8.3 | 9.2 | 9.5 |
| **5 mins after rinse** | 6.0 | 7.3 | 7.6 | 7.9 | 8.7 | 8.8 |
| **15 mins after rinse** | 5.5 | 6.6 | 7.2 | 7.4 | 7.9 | 8.3 |
| **30 mins after rinse** | 5.0 | 6.2 | 6.4 | 6.9 | 6.3 | 6.9 |
| **45 mins after rinse** | 3.2 | 4.7 | 4.3 | 5.4 | 5.8 | 6.2 |

**TABLE 15**

| | **Perceived level of taste astringency** | | | | | |
|---|---|---|---|---|---|---|
| **Sample** | **K** | **L** | **M** | **N** | **O** | **P** |
| **At application** | 5.7 | 5.8 | 6.3 | 6.2 | 7.9 | 8.4 |
| **0 mins after rinse** | 5.4 | 5.2 | 6.1 | 6.0 | 7.5 | 8.1 |
| **5 mins after rinse** | 4.6 | 6.3 | 5.7 | 5.4 | 6.8 | 7.6 |
| **15 mins after rinse** | 4.2 | 5.4 | 5.1 | 4.8 | 6.4 | 7.2 |
| **30 mins after rinse** | 3.2 | 3.9 | 4.8 | 4.1 | 5.2 | 6.7 |
| **45 mins after rinse** | 2.1 | 3.3 | 4.1 | 3.7 | 4.6 | 5.2 |

As shown in TABLES 14 and 15, the perceived intensity of both taste sensations increases when the propolis concentration is higher. The perceived taste sensations did not reduce significantly 0 minutes after panelists rinsed their mouth with water. A significant reduction was noted between the 15-minute and 30-minute time points.

### EXAMPLE 11

For EXAMPLE 11, an evaluation of the perceived effectiveness of the sample formulations K to P was conducted. The same panel of trained sensory experts (as per TABLES 14 and 15), who at the time reported discomfort due to cold symptoms including a sore throat, separately applied 3 sprays (0.45ml) of each propolis composition (K-P) to the back of their throat a second time and rated them on a 10-point scale according to the perceived level of effectiveness for improving symptoms of a sore throat, from 0 ('Not Effective') to 10 ('Very Effective'). Evaluations were conducted 30 minutes and 1 hour after application. In this test, a numerical score between 3.5 and 6.5 indicates an acceptable level of perceived effectiveness, which is not statistically significant. Numerical scores above 6.5 indicate that the level of perceived effectiveness is high, whereas scores below 3.5 suggest a low level of perceived effectiveness. Results are shown in TABLE 16 below.

**TABLE 16**

| | **Perceived level of effectiveness for improving symptoms of a sore throat** | | | | | |
|---|---|---|---|---|---|---|
| **Sample** | **K** | **L** | **M** | **N** | **O** | **P** |
| **30 minutes after application** | 7.9 | 7.9 | 8.4 | 8.5 | 8.8 | 8.8 |
| **1 hour after application** | 9.1 | 8.7 | 9.2 | 9.4 | 9.8 | 9.4 |

All scores reported by the panelists in TABLE 16 for all samples K to P are above 6.5, which indicates that the perceived level of effectiveness is high in general and not negatively impacted by the perceived taste sensations of bitterness and astringency. As such, the sensory acceptability of the samples was not correlated to their perceived effectiveness; even if the taste was not found to be acceptable to panelists, this did not modulate their perceptions of effectiveness. Nonetheless, the lack of sensory acceptability will reduce the commercial viability of propolis compositions.

### EXAMPLE 12

For TABLE 17, a panel of 18 trained sensory experts evaluated the taste acceptability of the compositions K to P on the 9-point hedonic taste scale developed by the Quartermaster Food and Container Institute of the U.S. Armed Forces. Panelists applied 3 sprays (0.45ml) of each composition (K-P) to the back of their throat and rated each sample separately, from 1 ('Dislike Extremely') to 9 ('Like Extremely') according to their personal perception of the taste acceptability of each sample. A single evaluation was conducted, which was immediately after product application. Results are shown in TABLE 17 below.

**TABLE 17**

| | **Perceived taste acceptability based on the 9-point hedonic taste scale** | | | | | |
|---|---|---|---|---|---|---|
| **Sample** | **K** | **L** | **M** | **N** | **O** | **P** |
| **After application** | 3.1 | 3.0 | 2.0 | 1.6 | 1.1 | 1.0 |

The data shown in TABLE 17 above indicate a low level of perceived taste acceptability for propolis compositions without a TAS2R antagonist, with an average score of 1.9. All scores in TABLE 17 are at or below the 3-point mark, which is 'Dislike Moderately'. For samples containing propolis concentrations between 15% and 60% by total weight of the composition, scores were at or below the 2-point mark, which is 'Dislike Very Much'. For samples containing propolis concentrations above 60% by total weight of the composition, scores were at the 1-point mark (Dislike Extremely), indicating the lowest taste acceptability. These results show that propolis compositions have low user acceptability due to their poor sensory profile, in line with extant research, which is likely to negatively impact their commercial viability.

TABLES 14, 15, 16 and 17 demonstrate the need to add bitter and astringent taste antagonists to propolis compositions, regardless of the concentration of propolis used.

### EXAMPLE 13

For EXAMPLE 13, all samples K1 to P6 had the same formulation as samples K to P (TABLE 13) but with the addition of a TAS2R antagonist. Each sample had sucralose added at 1% by total weight of the composition.

A panel of 15 trained sensory experts separately applied 3 sprays (0.45ml) of each propolis composition containing the TAS2R antagonist (K1-P6) to the back of their throat and rated them separately according to:
- the perceived level oftaste bitterness ofthe preparation by assigning a number between 0 ('Not Bitter') and 10 ('Very Bitter')
- the perceived level of taste astringency of the preparation by assigning a number between 0 ('Not Astringent') and 10 ('Very Astringent')

They then rinsed their mouth with 20ml of tap water at room temperature (average temperature of 20°C). Panelists assessed the perceived levels of taste astringency and bitterness according to the same two 10-point scales at 0 minutes after rinse expectoration, then again 5 minutes, 15 minutes, 30 minutes and 45 minutes after rinse expectoration. At each evaluation, they assessed each taste sensation separately on the 1 0-point scale. Further, at each evaluation, panelists were instructed to breathe in and out, and then to evaluate the overall taste sensations of bitterness and astringency. In this test, a numerical score between 4.0 and 7.0 indicates a noticeable trend in the specified parameter, which is not statistically significant, but noticeable. Numerical scores under 4.0 indicate significant differences or definite reductions in the perceived levels of taste bitterness and astringency. Results for both taste sensations are reported in TABLES 18 and 19 below.

**TABLE 18**

| | **Perceived level of taste bitterness for samples containing a TAS2R antagonist (1% sucralose)** | | | | | |
|---|---|---|---|---|---|---|
| **Sample** | **K1** | **L2** | **M3** | **N4** | **05** | **P6** |
| **At application** | 3.3 | 3.2 | 3.4 | 3.2 | 4.2 | 4.3 |
| **0 mins after rinse** | 2.2 | 2.6 | 3.1 | 2.9 | 3.4 | 3.6 |
| **5 mins after rinse** | 2.1 | 2.3 | 2.8 | 2.7 | 3.0 | 3.3 |
| **15 mins after rinse** | 1.7 | 1.9 | 2.4 | 2.2 | 2.6 | 2.8 |
| **30 mins after rinse** | 1.2 | 1.5 | 1.9 | 1.9 | 2.1 | 2.2 |
| **45 mins after rinse** | 0.3 | 1.1 | 1.2 | 1.1 | 1.5 | 1.7 |

**TABLE 19**

| | **Perceived level of taste astringency for samples containing a TAS2R antagonist (1% sucralose)** | | | | | |
|---|---|---|---|---|---|---|
| **Sample** | **K1** | **L2** | **M3** | **N4** | **O5** | **P6** |
| **At application** | 3.2 | 3.0 | 3.7 | 3.7 | 3.9 | 4.1 |
| **0 mins after rinse** | 3.0 | 2.4 | 3.1 | 3.4 | 3.5 | 3.8 |
| **5 mins after rinse** | 2.6 | 2.2 | 2.8 | 3.1 | 3.3 | 3.5 |
| **15 mins after rinse** | 2.2 | 1.9 | 2.4 | 2.8 | 2.9 | 3.1 |
| **30 mins after rinse** | 1.3 | 1.5 | 1.7 | 2.5 | 2.6 | 2.8 |
| **45 mins after rinse** | 0.4 | 0.7 | 1.2 | 1.9 | 2.2 | 2.5 |

As shown in TABLES 18 and 19, the taste sensations of bitterness and astringency were significantly reduced when a TAS2R antagonist (1% sucralose) was added. The reduction was noticeable across all sample formulations K1 to P6 and for both carrier materials used (propylene glycol and ethanol).

### EXAMPLE 14

For EXAMPLE 14, an evaluation of the perceived effectiveness of the sample formulations containing 1% sucralose was conducted. The same panelists (as per TABLES 18 and 19), who at the time reported discomfort due to cold symptoms including a sore throat, separately applied 3 sprays (0.45ml) of each propolis composition (K1-P6) to the back of their throat and rated them on a 10-point scale according to the perceived level of effectiveness, from 0 ('Not Effective') to 10 ('Very Effective'). Evaluations were conducted 30 minutes and 1 hour after application. In this test, a numerical score between 3.5 and 6.5 indicates an acceptable level of perceived effectiveness, which is not statistically significant. Numerical scores above 6.5 indicate that the level of perceived effectiveness is high, therefore reducing the taste sensations of bitterness and astringency does not impact users' perceived effectiveness of a propolis composition containing a TAS2R antagonist for improving the symptoms of a sore throat. Results are shown in TABLE 20 below.

**TABLE 20**

| | **Perceived level of effectiveness for improving symptoms of a sore throat for samples containing a TAS2R antagonist (1% sucralose)** | | | | | |
|---|---|---|---|---|---|---|
| **Sample** | **K1** | **L2** | **M3** | **N4** | **05** | **P6** |
| **30 minutes after application** | 7.8 | 7.9 | 8.5 | 8.3 | 8.7 | 8.7 |
| **1 hour after application** | 8.9 | 8.5 | 9.1 | 9.2 | 9.5 | 9.2 |

All scores reported by the panelists in TABLE 20 are above 6.5, which indicates that users' perception of the effectiveness of a propolis composition is not negatively impacted by reduced negative taste sensations of bitterness and astringency. There is not a significant difference between the samples containing a TAS2R antagonist and the samples without a TAS2R antagonist. Therefore, the user acceptability and commercial viability are not negatively impacted when the taste sensations of bitterness and astringency are reduced.

### EXAMPLE 15

For EXAMPLE 15, a panel of 18 trained sensory experts (as per TABLE 17) evaluated the taste acceptability of the sample compositions (K1 to P6) containing TAS2R receptor antagonist 1% sucralose on the 9-point hedonic taste scale developed by the Quartermaster Food and Container Institute of the U.S. Armed Forces. Panelists applied 3 sprays (0.45ml) of each propolis composition (K1-P6) to the back of their throat and rated each sample separately, from 1 ('Dislike Extremely') to 9 ('Like Extremely') according to their personal perception of the taste acceptability of each sample. A single evaluation was conducted after application. Results are shown in TABLE 21 below.

**TABLE 21**

| | **Perceived taste acceptability for samples containing a TAS2R antagonist (1% sucralose) based on the 9-point hedonic taste scale** | | | | | |
|---|---|---|---|---|---|---|
| **Sample** | **K1** | **L2** | **M3** | **N4** | **05** | **P6** |
| **After application** | 8.2 | 8.1 | 7.7 | 7.7 | 7.2 | 7.1 |

The data shown in TABLE 21 above indicate a considerable level of perceived taste acceptability for propolis compositions containing a TAS2R antagonist (1% sucralose). All scores were above the 7-point mark, which is 'Like Moderately'. For samples containing lower concentrations of propolis (5% by total weight of the composition), scores were above the 8-point mark, which is 'Like Very Much'.

For purposes of comparison, TABLE 22 below provides the taste acceptability scores for the samples without a TAS2R antagonist (K-P) and the samples with the TAS2R antagonist, 1% sucralose, added (K1-P6).

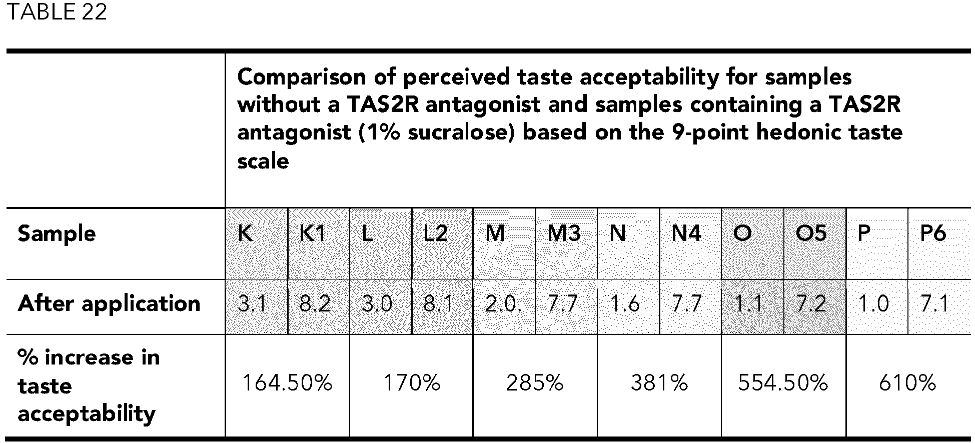

The results show that the addition of a TAS2R antagonist when a TAS2R agonist is present (polyphenols) will significantly improve the taste acceptability of propolis compositions, by 361% on average. The overall user acceptability and commercial viability of the propolis compositions of the present invention will therefore be significantly higher compared to compositions without a TAS2R antagonist.

TABLES 18 and 19 demonstrate that the present invention provides a practical and effective solution to improving the sensory acceptability of propolis compositions, thus increasing commercial viability. As was posited previously, the addition of a TAS2R antagonist in the presence of a TAS2R agonist (polyphenols) will reduce the perceived taste sensations of bitterness and astringency associated with propolis activation of this receptor without a negative impact on perceived effectiveness. TABLES 21 and 22 show an average improvement of 361% in user taste acceptability when a TAS2R antagonist is added compared to the samples without the antagonist. Further, TABLE 20 shows that the perceptions of effectiveness of propolis compositions of the present invention are not negatively impacted by a reduction in negative taste sensations. As such, the addition of a TAS2R antagonist to a propolis composition will benefit its user acceptability and commercial viability without diminishing its therapeutic benefits.

### REFERENCES

Amarowicz, R., Troszynska, A., Barylko-Pikielna, N. A. and Shahidi. F. (2004) 'Polyphenolics Extracts from Legume Seeds: Correlations Between Total Antioxidant Activity, Total Phenolics Content, Tannins Content and Astringency', Journal of Food Lipids, 1 1(4), pp. 278-286.
Ares, G., Barreiro, C., Deliza, R. and Gámbaro, A. (2009)'Alternativesto Reduce the Bitterness, Astringency and Characteristic Flavour of Antioxidant Extracts', Food Research International, 42(7), pp. 871-878.
De Castro, S. L. (2001) 'Propolis: Biological and Pharmacological Activities Therapeutic Uses of This Bee-Product', Annual Review of Biomedical, 3, pp. 49-83.
Grange, J. M. and Davey, R. W. (1990) 'Antibacterial Properties of Propolis (Bee Glue)', Journal of the Royal Society of Medicine, 83(3), pp. 159-160.
Kedzia, B. and Holderna-Kedzia, E. (2006) 'The Bee Products in the Nutrition and Supplementation', Postepy Fitoterapii, 4, pp. 213-221.
Khayyal, M. T., el-Ghazaly, M. A. and el-Khatib, A. S. (1993) 'Mechanisms Involved in the Antiinflammatory Effect of Propolis Extract', Drugs under Experimental and Clinical Research, 19(5), pp. 197-203.
Kocot, J., Kielczykowska, M., Luchowska-Kocot, D., Kurzepa, J., Musik, I. (2018) 'Antioxidant Potential of Propolis, Bee Pollen, and Royal Jelly: Possible Medical Application', Oxidative Medicine and Cellular Longevity. doi: 10.1155/2018/7074209.
Kurek-Górecka, A., Rzepecka-Stojko, A., Górecki, M., Stojko, J., Sosada, M. and Swierczek-Zieba, G. (2013) 'Structure and Antioxidant Activity of Polyphenols Derived from Propolis', Molecules, 19(1), pp. 78-101. doi: 10.3390/molecules19010078.
Lesschaeve, I. and Noble, A. C. (2005) 'Polyphenols: Factors Influencing Their Sensory Properties and Their Effects on Food and Beverage Preferences', American Journal of Clinical Nutrition, 81, pp. 330-335.
Magnavacca, A., Sangiovanni, E., Racagni, G. and Dell'Agli, M. (2022) 'The Antiviral and Immunomodulatory Activities of Propolis: An Update and Future Perspectives for Respiratory Diseases', Medical Research Review, 42(2), pp. 897-945.
Marcucci, M. (1995) 'Propolis: Chemical Composition, Biological Properties and Therapeutic Activity', Apidologie, 26(2), p 8399.
Meilgaard, M. C., Carr, B. T., and Civille, G. V. (2007) Sensory Evaluation Techniques. 4th edn. Boca Raton: CRC Press.
Park, Y. K., Alencar, S. M. and Aguiar, C. L. (2002) 'Botanical Origin and Chemical Composition of Brazilian Propolis', Journal of Agricultural and Food Chemistry, 50(9), pp. 2502-2506.
Pietta, P. G., Gardana, C. and Pietta, A. M. (2002) 'Analytical Methods for Quality Control of Propolis', Fitoterapia, 73(1), S7 - S20.
Pobiega, K., Kraśniewska, K. and Gniewosz, M. (2019)'Application of Propolis in Antimicrobial and Antioxidative Protection of Food Quality - A Review', Trends in Food Science & Technology, 83, pp. 53-62.
Rios, F., Lobo, M. and Samman, N. (2017) 'Acceptability of beehive products as ingredients in quinoa bars', Journal of the Science of Food and Agriculture, 98(1), pp. 174-182.
Scadding, G. K. (2018) 'A Taste of Things to Come?', The Journal of Allergy and Clinical Immunology: In Practice, 6, pp. 1081-1082.
Scheller, S., Wilczok, T., Imielski, S., Król, W., Gabryś, J. and Shani, J. (1989)_'Free Radical Scavenging by Ethanol Extract of Propolis', International Journal of Radiation Biology, 57, pp. 461-465. doi: 10.1080/09553009014552601.
Soares, S., Brandão, E., Guerreiro, C., Soares, S., Mateus, N. and de Freitas, V. (2020) 'Tannins in Food: Insights into the Molecular Perception of Astringency and Bitter Taste', Molecules, 25(11), p. 2590.
Soares, S., Silva, M. S., García-Estevez, I., Gro β mann, P., Brás, N., Brandão, E., Mateus, N., de Freitas, V., Behrens, M. and Meyerhof, W. (2018) 'Human Bitter Taste Receptors Are Activated by Different Classes of Polyphenols', 66(33), Journal of Agricultural and Food Chemistry, pp. 8814-8823.
Takahashi, S., Kurogi, M. and Saitoh, O. (2021) 'The Diversity in Sensitivity of TRPA1 and TRPV1 of Various Animals to Polyphenols', Biomedical Research, 42(2), pp. 43-51.
Wagh, V. D. (2013) 'Propolis: A Wonder Bees Product and Its Pharmacological Potentials', Advances in Pharmacological and Pharmaceutical Sciences, 2013.
Walker, P. and Crane, E. (1987) 'Constituents of Propolis, Apidologie, 18, pp. 327-334.
Wollenweber, E., Hausen, B. M. and Greenaway, W. (1990) 'Phenolic Constituents and Sensitizing Properties of Propolis, Poplar Balsam and Balsam of Peru', Bulletin de Liaison-Groupe Polyphenols, 15, pp. 112-120.

## Claims

1. A propolis composition comprising:
a) at least 5%, by weight of the propolis composition, of a propolis source (which can be raw propolis, propolis extract, or combinations thereof); and
b) at least one of an antagonist to TRPA1 receptor or TAS2R receptors.

2. The propolis composition of Claim 1, wherein the TRPA1 receptor antagonist is at least one of: Cinnamon bark oil; Vanillic acid; y -Methyl decalactone; Piperazine; Manganese chloride; Desoxycholic acid; Sodium erythorbate; DI-verbenone; 3-hydroxybenzoic acid; Ferric chloride; Phenylethyl isovalerate; Methyl linoleate; or Isobutyl isobutyrate.

3. The propolis composition of any of Claims 1 to 2, wherein the TRPA1 antagonist is present in an amount of from 0.02% to 3%, by total weight of the composition.

4. The propolis composition of any of Claims 1 to 2, wherein the TRPA1 antagonist is present in an amount of from 0.25% to 2.5%, by total weight of the composition.

5. The propolis composition of Claim 1, wherein the TAS2R receptors antagonist is at least one of: Sucralose; Probenecid (p-(dipropylsulfamoyl) benzoic acid; SBA; 3-Hydroxypropionic acid (3HP); 1,5-anhydro-3-O-methyl-D-mannitol (3HD); Sodium saccharin; Aspartame; Sorbitol; Xylitol; or Hardwickiic acid (as per WO2013072332A1).

6. The propolis composition of Claim 1, wherein the TAS2R receptors antagonist is at least one sweetener of: Sucralose; Sodium saccharin; Aspartame; Sorbitol; or Xylitol; preferably Sucralose.

7. The propolis composition of any of Claims 1, 5 or 6, wherein the TAS2R antagonist is present in an amount of from 0.05% to 2.5%, by total weight of the composition.

8. The propolis composition of any of Claims 1, 5 or 6, wherein the TAS2R antagonist is present in an amount of from 1% to 3%, by total weight of the composition.

9. The propolis composition of any of Claims 1 to 8, wherein the TRPA1 antagonist is present in an amount of from 0.02% to 3% and the TAS2R antagonist is present in an amount of from 0.05% to 2.5%, by weight of the composition.

10. The propolis composition of any of Claims 1 to 8, wherein the TRPA1 antagonist is present in an amount of from 0.25% to 2.5% and the TAS2R antagonist is present in an amount of from 1% to 3%, by weight of the composition.

11. A method of reducing the negative taste sensations of pungency, bitterness and astringency produced by the application of a propolis composition comprising:
a) providing a propolis composition comprising:
1) at least 5%, by weight of the propolis composition, of a propolis source (which can be raw propolis, propolis extract, or combinations thereof); and
2) at least one of an antagonist to TRPA1 receptor or TAS2R receptors.
b) contacting a body surface of the the oral cavity, and any surfaces or areas thereof, with the propolis composition.

12. The method of Claim 11, wherein the propolis composition is any of the propolis compositions of Claims 1 to 10.

13. The method of Claim 11, wherein the TRPA1 antagonist is any of the compounds of Claims 2 to 4, preferably Sucralose.

14. The method of Claim 11, wherein the TAS2R antagonist is any of the compounds of Claims 5 to 8.

15. The method of Claims 11 to 14, wherein the propolis composition has a taste acceptability score above 5 (Neither Like nor Dislike) on the 9-point hedonic taste scale.
